(19) **Europäisches Patentamt**

**European Patent Office**

**Office européen des brevets**

(11) **EP 0 642 796 B1**

(12) **EUROPÄISCHE PATENTSCHRIFT**

(45) Veröffentlichungstag und Bekanntmachung des Hinweises auf die Patenterteilung:
**05.12.2001 Patentblatt 2001/49**

(51) Int Cl.[7]: **A61K 39/112**, A61K 39/02, C12N 1/20
// (C12N1/20, C12R1:42)

(21) Anmeldenummer: **93114221.0**

(22) Anmeldetag: **04.09.1993**

(54) **Salmonella-Lebendimpfstoff**

Salmonella live vaccines

Vaccins de salmonella vivants

(84) Benannte Vertragsstaaten:
**AT BE CH DE DK ES FR GB GR IE IT LI LU NL PT SE**

(43) Veröffentlichungstag der Anmeldung:
**15.03.1995 Patentblatt 1995/11**

(73) Patentinhaber: **Lohmann Animal Health GmbH & Co. KG**
**27472 Cuxhaven (DE)**

(72) Erfinder:
• **Linde,Klaus Prof Dr**
**D-04279 Leipzig (DE)**
• **Beer,Jörg Dr**
**D-42777 Leipzig (DE)**
• **Randhagen,Bärbel Dipl-Ing**
**D-04277 Leipzig (DE)**

(74) Vertreter: **Siemons, Norbert, Dr.-Ing.**
**Patentanwälte Hauck, Graalfs, Wehnert, Döring, Siemons et al**
**Neuer Wall 41**
**20354 Hamburg (DE)**

(56) Entgegenhaltungen:
**EP-A- 0 263 528**

• **VACCINE Bd. 10, Nr. 5 , 1992 , GUILDFORD UK Seiten 337 - 340 LINDE K. ET AL 'Prophylaxis of Salmonella abortus ovis-induced abortion of sheep by a Salmonella typhimurium live vaccine'**
• **VACCINE Bd. 8, Nr. 3 , 1990 , GUILDFORD UK Seiten 278 - 282 LINDE K. ET AL 'Stable Salmonella vaccine strains with two or more attenuating mutations and any desired level of attenuation'**
• **JOURNAL OF BACTERIOLOGY Bd. 170, Nr. 9 , 1988 , BALTIMORE Seiten 3991 - 3995 EDWARDS, M.F. ET AL 'Construction of aro his pur strains of Salmonella typhi'**
• **JOURNAL OF CLINICAL MICROBIOLOGY Bd. 15, Nr. 6 , 1982 , WHASINGTON DC Seiten 1085 - 1091 HICKMAN F.W. ET AL 'Evaluation of two Salmonella typhi strains with reduced virulence for use in teaching and proficiency testing'**
• **THE MERK MANUAL OF DIAGNOSIS AND THERAPY, 1992, MERK RESEARCH LABORATORIES, Seiten 1085-1091**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung bezieht sich auf einen Salmonella-Lebendimpfstoff, hergestellt aus mindestens einem attenuierten, immunogenen Lebendimpfstamm, der zur Attenuierung zumindest eine Streptomycin- und eine Rifampicin-Resistenz Stoffwechseldrift-Mutation aufweist.

Die Mehrzahl der Salmonella-bedingten Gastroenteritiden des Menschen werden durch kontaminierte tierische Lebensmittel ausgelöst. Insbesondere Hühner bzw. Hühnereier, die mit dem derzeit prädominant auftretenden Serovar-Salmonella enteritidis infiziert sind, haben in jüngster Zeit verstärkt Infektionen hervorgerufen. Betroffen sind generell aber alle Lebensmittel, die von Tieren aus Massenhaltung stammen. Hierbei werden in der Regel viele Tiere auf engem Raum gehalten, was der Ausbreitung von Infektionen in den Tierbeständen Vorschub leistet.

Die Gefahr einer Übertragung der Infektion von dem infizierten Tier auf den Menschen läßt sich zum einen durch übliche veterinärmedizinische Maßnahmen zur Infektionskettenunterbrechung herabsetzen. Weiterhin kann eine sorgfältige Beachtung der küchenhygienischen Vorschriften bei Verarbeitung kontaminierter tierischer Lebensmittel eine Übertragung auf den Menschen verhindern. Gerade die letztgenannten Vorschriften werden jedoch bei der Lebensmittellagerung und Zubereitung nicht immer in ausreichendem Maße berücksichtigt. Es ist also erforderlich, von vornherein auszuschließen, daß infizierte Tiere verarbeitet werden. Dies läßt sich z.B. durch eine Impfung der Tierbestände gegen Salmonalla-Infektionen erreichen.

Geeignete Salmonalla-Lebendimpfstoffe müssen eine Reihe von unterschiedlichen Bedingungen erfüllen:

1. Die Virulenz der zur Herstellung der Impfstoffe eingesetzten Impfstämme muß so eingestellt sein, daß einerseits eine inapparente Infektion und andererseits eine ausreichende Persistenz der Impfstämme im Wirtsgewebe als Voraussetzung für eine hohe Immunogenität garantiert ist.

2. Es muß weitgehend sichergestellt sein, daß die eingesetzten Impfstämme bzgl. ihrer Virulenz und Schutzeigenschaften stabil sind, d.h. daß sie nicht zu dem virulenten Wildstamm rückmutieren.

3. Um die Wahrscheinlichkeit möglicher Infektketten herabzusetzen, sollte weiterhin vorgesehen werden, daß die Impfstämme nicht dauerhaft lebend ausgeschieden werden können bzw. in der Außenwelt nur kurze Zeit überleben können.

Auf die vorgenannten drei Bedingungen, die ein Lebendimpfstoff erfüllen muß, soll im folgenden detailliert eingegangen werden. Wie unter 1. ausgeführt basiert die Herstellung eines geeigneten Salmonella-Lebendimpfstoffes auf einer Reduktion der Virulenz (Attenuierung) der pathogenen Salmonellen bei gleichzeitigem Erhalt ihrer Antigenstrukturen und damit der immunogenen Wirkung im Wirt. Eine Möglichkeit besteht z.B. darin, als Impfstämme Deletionsmutanten, z.B. Pur- oder Aro-auxotrophe Klone einzusetzen. Der Attenuierungsgrad dieser Impfstämme hängt vom Metabolitenmangel in vivo ab, was möglicherweise eine Feinanpassung an den zu immunisierenden Wirt erschwert. Es wird in diesem Zusammenhang allerdings auf die EP 0 263 528 verwiesen, in der stabile Asp-Mutanten von Salmonella-typhimurium mit unterschiedlich hoher Virulenzreduktion beschrieben werden. Durch Auswahl geeigneter Asp-Mutanten kann man Impfstämme mit einer der jeweiligen Wirtsspezies angepaßten Attenuierungshöhe herstellen.

Eine weitere Möglichkeit der Attenuierung besteht darin, solche Impfstämme einzusetzen, deren Virulenzreduzierung auf eine Stoffwechseldrift-Mutation (im folgenden Stwd-Mutation oder Marker genannt) zurückzuführen ist. Der Begriff "Stoffwechseldrift" umfaßt alle durch Mutationen funktionell veränderten essentiellen Enzyme bzw. funktionell wichtigen Zellkompartimente, wie z.B. Ribosomenproteine, Gyrase, RNA-Polymerase, Permeasen, wobei als Folge dieser Mutationen die Funktionen der Translation, DNA-Replikation, -Transkiption oder Permeation mehr oder weniger ausgeprägt gestört sind. Solche Stwd-Mutanten weisen weiterhin eine Resistenz gegenüber speziellen Antibiotika oder anderen Substanzen (Noxen) auf. Auf besonders einfache Weise können Stwd-Mutanten im Labor als chromosomale Antibiotika-Resistenzmutanten erhalten werden. Aus der EP 0 263 528 sind in diesem Zusammenhang z.B. Stwd-Mutanten mit einer Resistenz gegen Nalidixinsäure (Nal), Streptomycin (Sm) oder Rifampicin (Rif) bekanntgeworden. Insbesondere bei Einbau mehrerer Stwd-Marker in einen Impfstamm (2- oder 3-Marker-Impfstamm) ergeben sich de facto unbegrenzte Möglichkeiten zur Herstellung jeder gewünschten einer speziellen Wirtsspezies optimal angepaßten Gesamtattenuierungshöhe.

Zum Stand der Technik "Attenuierung mittels Stwd-Mutationen" wird auf folgende Veröffentlichungen verwiesen: DD-WP 155 294; DD-WP 218 834; DD-WP 235 828; DD-WP 253 182, DD-WP 253 184; DD-WP 281 118; DD-WP 294 420; EP 0 263 528.

Eine weitere (oben unter 2. erwähnte) Bedingung ist, daß die durch Mutation gewonnenen attenuierten Impfstämme nicht zu dem virulenten Wildstamm zurückmutieren. Die geforderte Stabilität läßt sich einerseits dadurch erreichen, daß nur derartige Impfstämme eingesetzt werden, bei denen in vitro keine Reversionen nachweisbar sind, bzw. deren Reversionshäufigkeiten $<10^{-7}$ sind. Eine weitere Möglichkeit ist, solche Impfstämme einzusetzen, die mehrere voneinander unabhängig die Virulenz reduzierende Mutationen aufweisen. Hier ist die Wahrscheinlichkeit einer Rückmutation nahezu ausgeschlossen.

Die letzte, oben unter 3. im Zusammenhang mit dem Stichwort "Unterbrechung der Infektkettenbildung" angesprochene Bedingung betrifft insbesondere die Gefahren, die mit einer möglichen Ausscheidung und einem dauerhaften Überleben der Impfstämme außerhalb des geimpften Wirtes einhergehen. In diesem Zusammenhang ist es wünschenswert, die Ausscheidung und die Überlebensfähigkeit der Impfstämme in der Außenwelt zu reduzieren. Um eine ausreichende Immunantwort zu garantieren, darf andererseits jedoch die zeitweilige Überlebensfähigkeit der Impfstämme im Wirtsgewebe nach z.B. oraler oder parenteraler Applikation nicht oder nur geringfügig beeinträchtigt werden. Impfstammutanten, die derartigen Anforderungen genügen, sind z.B. aus der DD-WP 218 836, DD-WP 231 491, DD-WP 253 182, DD-WP 253 183, DD-WP 253 184 und EP 0 263 528 bekanntgeworden. Im Stand der Technik wird vorgeschlagen, geeignete Impfstämme zur Verringerung der Ausscheidung und ihrer Überlebensfähigkeit in der Außenwelt mit sogenannten antiepidemischen Markern zu optimieren. Der Begriff antiepidemische Marker bezeichnet im weiteren Sinne Outer-Envelope-Mutationen, die u.a. eine funktionelle Veränderung der Permeabilitäts-Barriere der Outer-Membrane bewirken.

Es stehen unterschiedliche antiepidemische Marker zur Verfügung, mit denen die Impfstämme in Abhängigkeit von der vorgesehenen Applikationsform versehen werden können. Die zur Zeit bekannten antiepidemischen Marker werden in Abhängigkeit von der Veränderung, die sie in der in der Outer-Membrane des Impfstammes bewirken, in drei Gruppen eingeteilt. Die erste Gruppe umfaßt die sogenannten antiepidemischen Hst-Marker. Der Einbau eines Hst-Markers bewirkt, daß ein Impfstamm hochsensibel gegenüber Galle, Aniondetergenzien, Makrolid-Antibiotika und anderen Noxen wird. Aufgrund der hohen Sensibilität gegenüber Galle kommt es zu einer verminderten Ausscheidung mit Fäces zurückzuführen auf die bereits im Darmlumen eintretende Inaktivierung der Impfstämme. Eventuell ausgeschiedene lebende Impfstammbakterien besitzen in der Außenwelt als Folge der fehlenden Permeabilitäts-Barriere in der Outer-Membrane gegen Tenside und Makrolide und anderen Noxen nur eine verkürzte Überlebenszeit. Die Bildung von Infektketten bei Verwendung von mit Hst-Markern versehenen Impfstämmen ist damit nahezu auszuschließen. Allerdings können mit Hst-Markern versehene Impfstämme zumindest bei Einsatz der üblichen Impfstoffdosen nur parenteral appliziert werden. Bei oraler Applikation wird die Virulenz aufgrund der hohen Gallesensibilität so stark beeinflußt, daß eine ausreichende immunogene Antwort nur bei Einsatz extrem hoher Impfstoffdosen erzielt werden kann. Für eine orale Applikation bietet es sich daher an, die Impfstämme mit einem antiepidemischen Marker aus einer der beiden weiteren bekannten Gruppen zu versehen. Unter die eine Gruppe fallen die sogenannten Rbt-Marker (reversion to bile tolerance). Der Rbt-Marker kann durch Mutation aus dem Hst-Marker gewonnen werden. Er verleiht dem Impfstamm ebenso wie der Hst-Marker eine antiepidemische Potenz. Im Unterschied zu dem Hst-Marker ist der mit einem Rbt-Marker versehene Impfstamm jedoch tolerant gegenüber Galle und kann daher ohne den Impfeffekt einschränkende Reduktion der Virulenz oral appliziert werden. Das gleiche gilt für die weitere Gruppe, die sogenannten Rtt-Marker (reversion to tenside tolerance). Der Rtt-Marker kann durch Mutation aus dem Rbt-Marker gewonnen werden. Der mit dem Rtt-Marker versehene Impfstamm ist tolerant gegenüber Tensiden und besitzt gleichzeitig aufgrund der verbleibenden hohen Sensibilität gegenüber Makroliden und anderen Noxen eine ausreichende antiepidemische Potenz. Auch der Rtt-Marker-Stamm kann problemlos oral appliziert werden.

Unter Zugrundelegen der vorgenannten Informationen und Veröffentlichungen lassen sich Lebendimpfstoffe herstellen, die alle erforderlichen Bedingungen erfüllen. Die Anpassung an den jeweiligen Wirt kann dabei z.B. durch Tierversuchsreihen erfolgen.

Es verbleibt ein weiteres Problem. Auch bei Beachtung aller Vorsichtsmaßnahmen ist nicht auszuschließen, daß z.B. mit der Impfung der Tiere oder der Herstellung der Impfstoffe befaßten Personen mit den zwar attenuierten aber nach wie vor pathogenen Salmonella-Impfstämmen in Berührung kommen. Bei gesunden Personen besteht kaum Gefahr, daß eine Infektion ausgelöst wird. Handelt es sich jedoch um Personen, deren Immunsystem geschwächt ist (z.B. im Falle einer HIV-Infektion), dann kann der Kontakt mit derartigen Impfstämmen zu einer Salmonelleninfektion führen.

Aufgabe der Erfindung ist es, weitere Salmonella-Lebendimpfstämme zur Herstellung eines Lebendimpfstoffes bereitzustellen.

Die Lösung dieser Aufgabe ist im Anspruch 1 angegeben.

Die Erfindung stellt erstmals einen Salmonella-Lebendimpfstoffe zur Verfügung, zu dessen Herstellung mindestens ein attenuierter Lebendimpfstamm eingesetzt wird, der mit einem Envelope-Marker versehen ist, der dem Impfstamm eine erhöhte Sensibilität gegenüber einem speziellen therapeutisch wirksamen Antibiotikum mit Ausnahme von Makroliden verleiht. Die zur Herstellung des erfindungsgemäßen Salmonella-Lebendimpfstoffes verwendeten Lebendimpfstämme sind also mit einem Envelope-Marker versehen, der ihnen in der Regel eine antiepidemische Potenz (Unterbrechung der Infektkettenbildung), ggf. eine Sensibilität gegenüber Makrolid-Antibiotika, jedenfalls aber eine erhöhte Sensibilität gegenüber einem anderen speziellen therapeutisch wirksamen Antibiotikum verleiht. Die Impfstämme lassen sich durch Einsatz des ausgewählten speziellen therapeutischen Antibiotikums relativ einfach nachweisen, so daß die Herstellung eines mit einem geeigneten Envelope-Marker versehenen Impfstammes keine größere Schwierigkeit darstellt. Es versteht sich, daß im Hinblick auf eine eventuell erforderliche Therapierbarkeit einer durch den Impfstoff hervorgerufenen ungewollten Infektion in erster Linie derartige Antibiotika ausgewählt werden, die eine gute Wirksamkeit gegenüber den eingesetzten Salmonella-Serovaren aufweisen.

Es versteht sich weiterhin, daß die ausgewählten Lebendimpfstämme von prädominanten Serovaren stammen.

Vorteilhafte Ausgestaltungen des erfindungsgemäßen Lebendimpfstoffes gemäß Anspruch 1 sind in den Unteransprüchen 2 bis 5 angegeben.

Wie oben bereits ausgeführt, gibt es unterschiedliche Möglichkeiten der Attenuierung von Lebendimpfstoffen. Eine Möglichkeit besteht darin, einen Auxotrophie-Marker (z.B. Asp⁻) in den Impfstamm einzubauen. Vorteilhafterweise wird der Impfstamm zur Attenuierung mit mindestens einer chromosomalen Antibiotika-Resistenzmutation versehen. Unter dem Begriff chromosomale Antibiotika-Resistenzmutation fallen im wesentlichen die eingangs beschriebenen Stwd-Marker. Es hat sich herausgestellt, daß durch Einsatz ausgewählter Stwd-Marker bzw. durch gezielte Kombination mehrerer derartiger Marker die Attenuierungshöhe des Impfstammes optimal einstellbar ist. Bei der Auswahl derartiger chromosomaler Antibiotika-Resistenzmutationen zur Attenuierung der Impfstämme muß jedoch darauf geachtet werden, daß diese die durch den Envelope-Marker bewirkte erhöhte Sensibilität gegenüber dem therapeutisch wirksamen Antibiotikum nicht ausschließt. Sinnvollerweise wird bei der Entwicklung des Impfstammes daher zunächst festgelegt, welches therapeutisch wirksame Antibiotikum gegebenenfalls zur Therapierbarkeit des Impfstammes eingesetzt werden soll. In Abhängigkeit davon wird dann der bzw. die chromosomale Antibiotika-Resistenzmutation zur Attenuierung des Impfstammes ausgewählt.

Der Envelope-Marker ist so ausgewählt, daß er dem Impfstamm (neben einer antiepidemischen Potenz und ggf. einer Makrolid-Sensibilität) eine erhöhte Sensibilität gegenüber einem Antibiotikum aus der Gruppe der Chinolone, Chloramphelicole oder Tetracycline verleiht. Als besonders vorteilhaft hat sich ein Envelope-Marker erwiesen, der dem Impfstamm eine erhöhte Sensibilität gegenüber dem zur Zeit gegen Salmonellen wirksamsten Antibiotikum Ciprofloxacin vermittelt. Insbesondere bei der Herstellung des letztgenannten Impfstammes ist vorteilhaft, das zur Attenuierung Stoffwechseldrift-Mutationen vorzusehen sind, die eine Streptomycin-und Rifampicin-Resistenz aufweisen. Diese Antibiotika-Resistenzen interferieren nicht mit einer möglichen Sensibilität des Impfstammes gegenüber dem Antibiotikum Ciprofloxacin.

Je nach gewünschtem Wirkungsspektrum kann der erfindungsgemäße Salmonella-Lebendimpfstoff aus einem oder mehreren Impfstämmen unterschiedlicher (prädominanter) Serovare der 0-Gruppen B (z.B. Salmonella typhimurium), D (z.B. Salmonella enteritidis), C (z.B. Salmonella infantis) und E (z.B. Salmonella anatum) als Mono-, Bi-, Tri- oder Tetra-Vakzin hergestellt werden. In diesem Zusammenhang wird auf die in Anspruch 4 angegebenen Vakzine verwiesen, die besonders geeignet sind.

Ein weiteres, bereits oben gestreiftes Problem besteht darin, daß aufgrund unterschiedlicher Empfänglichkeiten für jeden Wirt ein spezieller, angepaßter Lebendimpfstoff vorgesehen werden muß. In diesem Zusammenhang wird auf den bereits im Handel erhältlichen Salmonella typhimurium-Impfstoff Zoosaloral "Dessau" verwiesen, der bei einmaliger oraler Gabe Kälber ausreichend immunisiert, Hühner dagegen erst nach dreimaliger oraler Impfung gegen eine i.p. Toxoinfektion schützt (Linde, K. et al.: Vaccine 1990, 8, 278-282). Bezüglich der Wirtsspezies-abhängigen Empfänglichkeit für Salmonella typhimurium ergibt sich in etwa die Rangfolge Mäuse > Kälber > Hühner. Daraus folgt, daß z.B. Salmonella typhimurium-Impfstämme für Küken oder Hühner zur Kompensation der geringeren Empfänglichkeit einen (im Vergleich zu Mäusen) geringeren Attenuierungsgrad besitzen müssen. Es kann davon ausgegangen werden, daß dies sinngemäß auch für andere Salmonella-Serovare mehr oder weniger zutrifft.

Es hat sich herausgestellt, daß die Generationszeiten von Salmonella-Lebendimpfstämmen i.d.R. mit ihrer Attenuierungshöhe korrelierbar sind. Diese Korrelation zwischen Generationszeit und Attenuierungshöhe tritt insbesondere dann ein, wenn die Impfstämme zur Attenuierung mit einer chromosomalen Resistenzmutation versehen sind.

Das Verhältnis zwischen Generationszeit und Attenuierungsgrad erlaubt es, relativ zuverlässig für einen speziellen Wirt geeignete Impfstämme auch ohne vorherige Tierversuche auszuwählen. In diesem Zusammenhang schlägt Anspruch 5 einen zur Immunisierung von Küken und Hühnern geeigneten Lebendimpfstoff vor, der aus mindestens einem attenuierten Impfstamm hergestellt ist, dessen Generationszeit in etwa zwischen 29 bis 34 Minuten beträgt. Impfstämme mit solchen Generationszeiten weisen einen die geringe Empfindlichkeit von Küken/Hühnern für z.B. Salmonella typhimurium und andere Salmonella-Serovare kompensierenden niedrigeren (im Vergleich zu Impfstämmen für Kälber oder Mäuse) Attenuierungsgrad auf.

Wie eingangs bereits ausgeführt, ist ein effektiver Salmonella-Impfstoff insbesondere im Hinblick auf die Wirtsspezies Küken/Hühner interessant. Den bislang hinterlegten/ publizierten Salmonella typhimurium Stwd-Mutanten fehlt ein direkter Bezug zu dieser Wirtsspezies. Gemäß den Ansprüchen 4 und 5 werden nun Impfstoffe vorgestellt, deren Impfstämme im Hinblick auf Küken/Hühner optimal attenuierbar oder bereits attenuiert sind. Es wird in diesem Zusammenhang der Impfstamm S.tm Nal 2/Rif-9/Rtt hervorgehoben, der optimal für Küken/Hühner attenuiert ist. Das gleiche gilt für einen Impfstamm S.tm Nal 2/Rif 9, der im Zusammenhang mit dieser Anmeldung nicht hinterlegt worden ist, aber dennoch Erwähnung finden soll. Die zitierten Impfstämme haben eine Generationszeit von etwa 32 Minuten. Aus dieser Tatsache wurde das "Attenuierungs-Äquivalent Generationszeit" für die Auswahl optimal attenuierter anderer Stämme des gleichen oder der anderen Serovare abgeleitet. Allerdings werden die bei der Auswahl von für Hühner/ Küken geeigneter Impfstämme geeigneten Generationszeiten über einen Bereich von 29 bis 34 Minuten gefächert. Diese Fächerung entspricht der Tatsache, daß die Küken/ Hühner eine unterschiedliche Empfänglichkeit für differente Stämme bzw. stamm-abhängige Virulenzunterschiede der jeweiligen Serovare aufweisen können. Mit wenigen Versuchsreihen lassen sich aus vorselektierten Impfstämmen, die Generationszeiten zwischen 29 bis 34 Minuten aufweisen, diejenigen selektieren die optimal für Küken/Hühner attenuiert sind. Vorteilhafterweise können so zumindest die ansonsten für die Vorselektion erforderlichen Tierversuche entfallen.

Weiterhin ist es auch insbesondere im Hinblick auf den möglichen Wirt Küken/Hühner interessant, wenn, wie erfindungsgemäß vorgeschlagen, ein Salmonella-Lebendimpfstoff eingesetzt wird, der einen Impfstamm mit erhöhter Sensibilität gegenüber einem speziellen therapeutischen wirksamen Antibiotikum enthält. Hühner sind nämlich im Vergleich zu anderen Wirtsspezies z.B. Menschen, Kälber oder Ferkel, relativ kurzlebig. In der Regel werden sie nach einer vergleichsweise kurzen Aufzuchtphase geschlachtet und kommen als Gefrier- oder Frischfleisch in den Verkauf. Da die Impfung zumeist noch nicht sehr lange zurückliegt, ist davon auszugehen, daß zum Zeitpunkt der Schlachtung in den Hühnern noch lebende Salmonella-Impfstammbakterien vorhanden sein können. Diese können dann in die Außenwelt gelangen. Für die Normalpopulation sind die in Frage kommenden geringe Keimzahlen der stabil attenuierten Impfstämme bedeutungslos. Hier kann also die Gefahr einer Infektkettenbildung nahezu ausgeschlossen werden. Allerdings können in Einzelfällen auch abwehrgeschwächte Risikopatienten (z.B. HIV-Personenkreis) infiziert werden und mit klinischen Symptomen reagieren. Insbesondere im Hinblick auf diesen Personenkreis ist es erforderlich, daß eine durch den Impfstamm hervorgerufene Infektion rasch und unproblematisch unter Kontrolle gebracht werden kann. Im Hinblick darauf ist der Einbau einer Sensibilität gegenüber therapeutisch wirksamen Antibiotika als Sicherheits- und Therapiemarker somit eine sinnvolle, alle theoretischen Bedenken ausschließende Alternative.

Der Prototyp eines derartigen Sicherheits- und Therapiemarkers ist der sogenannte Ssq-Marker. Mit dem Ssq-Marker versehene Impfstämme besitzen eine Supersensibilität gegenüber Chinolonen, insbesondere gegenüber Ciprofloxacin, das das zur Zeit gegenüber Salmonellen wirksamste Antibiotikum darstellt. Auch der Ssq-Marker ist wie die eingangs beschriebenen Hst-, Rbt- und Rtt-Mutationen eine Envelope-Mutante und besitzt damit gleichfalls - in Abhängigkeit des jeweiligen Ssq-Markers von seiner Galle- und Anionen-Detergent-Toleranz bzw. Sensibilität - eine mehr oder weniger ausgeprägte antiepidemische Potenz.

Die Erfindung beschränkt sich nicht nur auf die Schaffung von besonders sicheren Lebendimpfstoffen. Ein weiteres Ziel besteht darin, ein Verfahren zur Herstellung eines im Hinblick auf einen speziellen Wirt optimal attenuierten Lebendimpfstoffes anzugeben, das weitgehend ohne Tierversuche auskommt.

Dieses Ziel wird mit einem Verfahren erreicht,dessen Prinzip darauf beruht, daß eine Attenuierung von Impfstämmen (insbesondere beim Einbau von Stwd-Markern) zu einer Verlängerung der Generationszeiten gegenüber dem Wildtyp führt. Es wurde bereits weiter oben ausgeführt, daß die verlängerten Generationszeiten einen Rückschluß auf den Attenuierungsgrad der Impfstämme erlauben können. Nach dem erfindungsgemäßen Verfahren reicht es daher aus, wenn einmalig (z.B. in einem Tierversuch) die bestimmte verlängerte Generationszeit eines für einen Wirt geeigneten Impfstammes ermittelt wird. Die ermittelte verlängerte Generationszeit kann dann als Richtwert bei der späteren Selektion weiterer Impfstämme (vom gleichen Serotyp) eingesetzt werden oder sogar als Attenuierungs-Äquivalent auf andere Serotypen der gleichen Gattung übertragen werden.

Zur Herstellung eines optimal auf Küken/Hühner abgestimmten Impfstoffes wird z.B. ein Impfstamm ausgewählt, dessen Generationszeit gegenüber dem Wildstamm (22 Minuten) auf etwa 29 bis 34 Minuten verlängert ist. Der eingesetzte Impfstamm kann dabei z.B. aus einem Wildstamm erhalten werden, der zunächst mit einer Streptomycin (Sm)- oder Nalidixinsäure (Nal)-Markierung versehen wird. Dadurch wird eine Generationszeitverlängerung von 22 auf 25 bis 29 Minuten bewirkt. Anschließend wird als weiterer, die Generationszeit verlängernder Marker ein Rifampicin (Rif)-Marker eingebaut.

Gemäß der aufgeführten wesentlichen Merkmale des Verfahrens kann ein Impfstoff hergestellt werden, in dem man

- im ersten Schritt von Salmonella-Wildstämmen Stwd-Mutanten eines bestimmten Antibiotika-Resistenz Phänotyps mit um drei bis sechs Minuten verlängerter Generationszeit isoliert, zwecks Herstellung von Stämmen mit geringer bis mäßiger Attenuierung,
- im zweiten Schritt von solchen gering bis mäßig attenuierten Einmarker-Stämmen erneut Stwd-Mutanten eines anderen Antibiotika-Resistenz-Phänotyps mit wiederum um zusätzlich drei bis sechs Minuten verlängerter Generationszeit isoliert, wodurch man einen Set von ImpfstammKandidaten mit abgestuft verlängerten Generationszeiten von etwa 29 bis 34 (Wildstamm etwa 22) Minuten erhält,
- was bei S.tm am i.p. Mäusemodell (da der Logarithmus der $LD_{50}$ linear mit der (verlängerten) Generationszeit korreliert) abgestuften $LD_{50}$-Werten von etwa $10^5$ bis $10^7$ (Wildstamm etwa $10^1$) cfu entspricht -, dann mit den einzelnen S.tm Impfstämmen dieses Sets ≤ 36 Stunden alte Küken einmal oral mit $10^9$ cfu immunisiert, nach zwei Wochen die immunisierten Tiere oral mit $10^6$ cfu Wildstamm infiziert; und nunmehr im Hinblick auf die Wechselbeziehung "maximal mögliche(r) Generationszeit-Verlängerung/ Attenuierungsgrad bei noch bestehender optimaler Reduktion der Wildstamm-Ausscheidung" den S.tm Nal 2/Rif 9 mit einer (verlängerten) Generationszeit von etwa 32 Minuten (und einer i.p. $LD_{50}$ Maus von etwa $10^6$ cfu) als Prototyp-Impfstamm favorisiert, zwecks Festlegung des "Attenuierungs-Äquivalenz (verlängerte) Generationszeit" für andere Stämme des gleichen Serovars bzw. Serovare mit nur mäßiger oder fehlender Virulenz für Mäuse.

- im dritten Schritt zur Impfstamm-Optimierung/-Akzeptanz-Erhöhung in die mittels Stwd-Mutation attenuierten Zweimarker-Impfstämme einen Ssq "Sicherheits-/und Therapie"-Marker einbaut, welcher die Sensibilität für Ciprofloxacin (Chloramphenicol, Doxycyclin, etc.) um etwa das Vierfache erhöht und gleichzeitig geringgradig die Ausscheidung und Überlebensfähigkeit in der Außenwelt reduziert.

Die angegebene Reihenfolge kann beliebig variiert und auch auf Noxen-Resistenz-Phänotypen: DD-WP 235 828 zurückgegriffen werden.

Die Erfindung betrifft schließlich auch noch spezielle Salmonella-Impfstämme, die gemäß Anspruch 20 Generationszeiten von 31 bis 32 Minuten aufweisen sowie Varianten dieser Impfstämme, die höher bzw. geringerer attenuiert sein können und Generationszeiten zwischen 29 und 34 Minuten aufweisen. Anspruch 22 schließlich betrifft die Verwendung derartiger Impfstämme zur oralen Immunisierung von Küken sowie oralen oder parenteralen Immunisierung von Hühnern gegen Salmonella-Infektionen.

Die in den Ansprüchen und nachfolgenden Beispielen aufgeführten (sowie hinterlegten) Salmonella-Impfstämme (mit und ohne Ssq-Marker) stehen als Beispiel für alle nach gleichem Prinzip herstellbaren Impfstammkandidaten mit abgestuften Attenuierungsgraden. Sie sind zur Herstellung immunogener Lebendimpfstoffe, insbesondere für Küken/Hühner nach an sich bekannten Vermehrungsverfahren geeignet. Bezüglich der speziell angegebenen mit Ssq-Marker versehenen Impfstämme werden Generationszeiten von etwa 32 Minuten angegeben. Diese Generationszeitangabe ist nicht als einengende Begrenzung aufzufassen. Bei stamm-abhängiger Virulenz (Invasivität/Kolonisierungs-Aktivitäts)-Unterschieden sind ggf. auch Generationszeiten von 29 bis 34 Minuten als Attenuierungs-Äquivalent denkbar.

In der nachfolgenden Tabelle werden die für die unterschiedlichen Serovare favorisierten Salmonella-Impfstämme noch einmal aufgeführt.

| Salmonella Impfstämme Serovar | Klon | Labor-Nummer | Hinterlegungs-Nummer |
|---|---|---|---|
| typhimurium | Ssq/Sm 60/Rif 42 | 4242 | DSM 8433 |
| enteritidis | Ssq/Sm 24/Rif 12 | 4266 | DSM 8435 |
| infantis | Ssq/Sm 153/Rif 7 | 4289 | DSM 8434 |
| anatum | Ssq/Sm 81/Rif 21 | 4279 | DSM 8441 |
| typhimurium | Nal 2/Rif 9/ Rtt | 4223 | DSM 8432 |

**) siehe Seite 18 Die Erfindung soll im folgenden anhand mehrerer Ausführungsbeispiele näher erläutert werden.

**)

Die Mikroorganismen wurden hinterlegt bei:
DSM-Deutsche Sammlung von
Mikroorganismen und Zellkulturen GmbH
Mascheroder Weg 1 B
D-38124 Braunschweig

## Ausführungsbeispiele

## Material und Methoden

### Verwendete Stämme

- Wildstämme

  . S.typhimurium (S.tm) 415 (Metschnikov-Institut, Moskau) i.p. $LD_{50}$ Maus $\leq 10^1$ cfu, Generationszeit etwa

22 Minuten

. S.enteritidis (S.ent) 318 (Prof. Selbitz, Universität Leipzig) i.p. LD$_{50}$ Maus etwa 10$^{5.0}$ cfu, Generationszeit etwa 22 Minuten

. S.infantis (S.inf) (Dr. Beer, Veterinär-Untersuchungsamt Chemnitz) Generationszeit etwa 22 Minuten

. S.anatum (S.ana) (Dr. Beer, Veterinär-Untersuchungsamt Chemnitz) Generationszeit etwa 22 Minuten

- Wildstämme mit neutraler Nalidixinsäure- und Streptomycin-Resistenzmarkierung für den Nachweis der reduzierten Ausscheidung bei immunisierten Kücken/Hühnern

. S.tm Nal/Sm, Generationszeit etwa 22,5 Minuten

. S.ent Nal/Sm, Generationszeit etwa 22,5 Minuten

. S.inf Nal/Sm, Generationszeit etwa 22,5 Minuten

. S.ana Nal/Sm, Generationszeit etwa 22,5 Minuten

- Impfstämme als Beispiel auch für andere Zwei- oder Dreimarker-Mutanten mit geringerer (oder höherer) Attenuierung / entsprechend weniger oder mehr verlängerter Generationszeit

. S.tm Ssq/Sm 60/Rif 42, Generationszeit etwa 31 Minuten Labor-Nr. 4242; Hinterlegungs-Nr. DSM 8433
. S.ent Ssq/Sm 24/Rif 12, Generationszeit etwa 32 Minuten Labor-Nr. 4266; Hinterlegungs-Nr. DSM 8435
. S.inf Ssq/Sm 153/Rif 7, Generationszeit etwa 31 Minuten Labor-Nr. 4289; Hinterlegungs-Nr. DSM 8434
. S.ana Ssq/Sm 81/Rif 21, Generationszeit etwa 32 Minuten Labor-Nr. 4279; Hinterlegungs-Nr. DSM 8441
. S. tm Nal 2/Rif 9/ Rtt, Generationszeit etwa 32 Minuten Labor-Nr. 4223; Hinterlegungs-Nr. DSM 8432 als Prototyp-Impfstamm (mit optimaler Attenuierung für Kücken/Hühner) zur Festlegung des "Attenuierungs-Äquivalenz (verlängerte) Generationszeit".

Nährmedien

- Nähragar (SIFIN, Berlin-Weißensee)
- Tryptose phosphate broth (Difco, USA)

Antibiotika

- Nalidixinsäure (CHINOIN, Budapest), Wildstämme MHK 6,2 μg/ml
- Streptomycin (Jenapharm ), Wildstämme MHK 6,2 μg/ml
- Rifampicin (UBM, Bukarest ), Wildstämme MHK 12,5 μg/ml
- Ciprofloxacin (Bayer ), Wildstämme MHK 0,05 μg/ml
- Chloramphenicol (Berlin-Chemie ), Wildstämme MHK 2,0 μg/ml
- Doxycyclin (Jenapharm ), Wildstämme MHK 4,0 μg/ml
- Erythromycin (Abbott ), Wildstämme MHK 60,0 μg/ml)

Versuchstiere und Haltungsbedingungen

Hähnchenkücken von Brauneilegern aus verschiedenen Brütereien wurden in Käfigen zu 5 - 10 Tieren gehalten und mit Putenaufzuchtfutter sowie Wasser ad libitum gefüttert.

Orale Immunisierung

Gruppen zu je 10 Kücken erhielten innerhalb 36 Stunden nach dem Schlüpfen oder (zum Zwecke des Vergleiches und Ermittlung des optimalen Immunisierungsalters) am vierten Lebenstag einmal oral $10^9$ cfu, teilweise $10^8$ cfu, des jeweiligen Impfstammes in den Schlund appliziert. Die Immunisierung unter Praxisbedingungen ist auch über das Trinkwasser möglich. (Nach vorausgegangenem Wasserentzug für 4 Stunden wird eine Trinkwassermenge von 2 ml/Kücken binnen 3 Stunden aufgenommen.

Orale Infektion

Zwei bis vier Wochen nach der oralen Immunisierung erhielten die Kücken oral mittels Pipette $10^6$ (oder $10^7$) cfu des jeweiligen neutral markierten homologen Wildstammes.

Nachweis der Ausscheidung des/der

- Impfstämme S.tm Ssq/Sm 60/Rif 42, S.ent Ssq/Sm 24/Rif 12, S.inf Ssq/ Sm 153/Rif 7 und S.ana Ssq/Sm 81/Rif 21: Nährmedien mit 100 µg Rifampicin und 200 µg Streptomycin/ml
- Impfstammes S.tm Nal 2/Rif 9 ohne und mit Rtt-Marker: Nährmedien mit 100 µg Rifampicin und 12,5 µg Nalidixinsäure/ml
- neutral Nal/Sm markierte Wildstämme: Nährmedien mit 100 µg Nalidixinsäure und 200 µg Streptomycin/ml Pro Kückengruppe und Untersuchungstag wurden jeweils fünf frische Kotproben in 2 ml physiologischer Kochsalzlösung suspendiert und zusätzlich die Verdünnungen $10^{-1}$ bis $10^{-4}$ hergestellt.
- Quantitative Bestimmung: Von der Originalsuspension und den Verdünnungen wurden 0,1 ml auf Nähragar mit je 1 % Lactose und Saccharose, 0,015 % Bromthymolblau (Bestimmung der Coli/Enterobakterien-Keimzahlen) sowie parallel auf das gleiche Medium mit den entsprechenden Antibiotika ausgespatelt. Als quantitatives Maß für die Ausscheidung bzw. die bei immunisierten Kücken im Vergleich zu den Kontrollen eingetretene Reduktion der Salmonella-Kolonisierung, wurde die Zahl der Salmonella-Kolonien zur Zahl der Enterobakterien-Kolonien als Promille-Wert berechnet.
- Qualitative Bestimmung: Der verbleibenden Originalsuspension wurde Antibiotika-Bouillon zugesetzt. Nach 24 Stunden 37°C Bebrütung erfolgte die Abimpfung auf Nähragar mit den jeweiligen Antibiotika-Zusätzen.

Die Bestätigung der angezüchteten Salmonellen erfolgte serologisch, biochemisch und durch Bestimmung der Marker.
Als quantitatives Maß für die Ausscheidung bzw. die bei immunisierten Kücken im Vergleich zu den Kontrollen eingetretene Reduktion der Salmonella-Kolonisierung, wurde die Zahl der Salmonellen-Kolonien zur Zahl der Enterobakterien-Kolonien als Promille-Wert berechnet.

**Beispiel 1**

**S.tm: Isolierung von spontanen chromosomalen Antibiotika-Resistenz-Klo nen als** (Nal-Stwd Ein- und) **Nal/Rif-Stwd Zweimarker-Impfstämme mit abgestuft verlängerten Generationszeiten zwischen etwa 29 bis 34** (Wildstamm etwa 22) **Minuten zwecks Isolierung eines Prototyp-Impfstammes** für **die Festlegung des "Attenuierungs-Äquivalenz** (verlängerte) **Generationszeit" für** Stämme des gleichen Serovars bzw. **Serovare mit nur mäßiger** (S. ent) **oder fehlender** (S.inf und S.ana) **Mäusevirulenz**

$10^9$ (und $10^{10}$) cfu des Wildstammes werden auf Nähragar mit 100 µg (oder in zwei Schritten über 50 µg und nachfolgend 400 µg) Nalidixinsäure/ml gespatelt und etwa zwei Tage bei 37°C incubiert. Die Resistenz-Klone werden über Nähragar passagiert, auf erhaltene Resistenz kontrolliert und im Vergleich zum Wildstamm die weniger oder mehr verminderte Extinktion (Spekol mit Röhrchenansatz, Zeiss-Jena, Wellenlänge 650 nm, Startkeimzahl $10^7$ cfu, Bebrütung im Schüttel-Wasserbad für 3 Stunden bei 37°C) bestimmt. Von geeignet erscheinenden Klonen wird im Abbott MS-2 Testsystem die Generationszeit gemessen. Vom Klon Nal 2 mit einer Generationszeit von etwa 28 (Wildstamm etwa 22) Minuten werden, wie oben beschrieben, auf Nähragar mit 400 µg Rifampicin/ml Resistenz-Klone gewonnen, von diesen Generationszeit bestimmt und die Nal 2/Rif-Klone mit abgestuft verlängerten Generationszeiten zwischen etwa 29 und 34 Minuten für die Festlegung des Richtwertes (verlängerte) "Generationszeit als Attenuierungs-Äquivalent" herangezogen (siehe Beispiel 3).

**Beispiel 2**

**Nachweis erhaltener Kolonisierungsaktivität der neutral Nal/Sm markierten S.tm, S.ent, S.inf und S.ana Wildstämme bei 17 bis 25 Tage alten Kücken**

Kücken im Alter von 17 bis 25 Tage erhielten oral mittels Pipette $10^6$ ($10^7$) cfu der neutral markierten Wildstämme. Über einen Zeitraum von zehn bis 15 Tagen wurde die quantitative Salmonella-Kolonisierungsdichte im Vergleich zur Enterobakterien-Keimzahl -Keimzahl ermittelt.

Bei dem gewählten Infektionsmodell (Dosis $10^6$ bis $10^7$ cfu) erfolgt nach oraler Infektion entweder bereits nach 24 Stunden die Ausscheidung der neutral markierten Wildstämme mit hohen Keimzahlen oder die Salmonella-Keimzahlen erreichen schrittweise ihre höchsten Werte im Kot erst zwischen dem dritten und sechsten Tag (Maximalwerte bei 50 Promille-Anteil an der Gesamt-Enterobakterien-Flora). Nach acht bis zehn Tagen fällt die Salmonella-Koloniezahl auf einen 1,0 bis 0,1 Promille-Anteil der Enterobakterien-Flora ab und bleibt in dieser Größenordnung über eine längere Zeit bestehen.

Diese Kolonisierungs-Dynamik beweist, daß die neutral Nal/Sm markierten Wildstämme zur Erfassung einer reduzierten Wildstamm-Kolonisierung bei immunisierten Kücken geeignet sind.

**Beispiel 3**

**Festlegung des S.tm Nal 2/Rif-Prototyp-Impfstammes mit optimal/em (verlängerter) Generationszeit/Attenuierungsgrad für Kücken/Hühner an Hand der Relation maximal mögliche(r) Generationszeit-Verlängerung/Attenuierungsgrad bei noch optimaler Reduktion der Wildstamm-Ausscheidung, zwecks Ermittlung des "Attenuierungs-Äquivalenz** (verlängerte) **Generationszeit"** für Stämme des gleichen Serovars bzw. **Serovare mit mäßiger oder fehlender Virulenz für Mäuse**

Kücken im Alter von ≤ 36 Stunden wurden einmal oral mit $10^9$ cfu der verschiedenen Stämme aus dem Set von S.tm Zweimarker-Impfstämmen mit zwischen 29 und 34 Minuten abgestuften Generationszeiten immunisiert und nach zwei Wochen oral mit $10^6$ cfu des Wildstammes belastet. Parallel wurden Kontroll-Kücken gleichen Alters oral infiziert.

Im Vergleich zu den Kontrollen scheiden die immunisierten Kücken, welche die Impfstämme mit abgestuften Generationszeiten zwischen 29 bis 32 Minuten erhielten, den Wildstamm, insbesondere in den ersten fünf bis zehn Tagen nach dem oralen Challenge, deutlich vermindert aus (gemessen am Promille-Anteil der Enterobakterien-Besiedlungsdichte). Die Differenzen betragen (mit der Zeit abnehmend) bis zu zwei Zehnerpotenzen. Ab sechsten bis zehnten Tag nach der oralen Belastung gleichen sich die Salmonellen-Besiedlungsdichten bei immunisierten Kücken denen von den Kontrollen (im 0,1 Promille-Bereich der Enterobakterien-Keimzahlen) an.

In Einzelfällen zeigen allerdings immunisierte Kücken eine verminderte Ausscheidung in der Größenordnung von einer log-Stufe auch noch nach dem zehnten Tag.

Bei Impfstämmen mit Generationszeiten ≥ 33 Minuten ist die Wildstamm-Ausscheidung vergleichsweise geringer reduziert, wobei die Differenz zu den Kontrollen in der Regel eine Zehnerpotenz nicht überschreitet.

Auf Grundlage der Relation: "Maximal mögliche(r) Generationszeit-Verlängerung/Attenuierungsgrad bei noch optimaler Reduktion der Wildstamm-Ausscheidung", wurde der S.tm Nal 2/Rif 9 mit einer Generationszeit von etwa 32 Minuten (und einer i.p. $LD_{50}$ Maus von etwa $10^6$ cfu) als Prototyp-Impfstamm ermittelt und seine (verlängerte) Generationszeit von etwa 32 Minuten als Richtwert "Attenuierungs-Äquivalent" eingesetzt.

**Beispiel 4**

**Erkennung/Nachweis des für Kücken/Hühner optimal attenuierten Salmonella typhimurium Klons S.tm Nal 2/Rif 9 (i.p. $LD_{50}$ Maus etwa $10^{6.0}$ (Wildstamm etwa $10^1$) cfu; Generationszeit als Attenuierungs-Äquivalent von etwa 22 auf 32 Minuten verlängert) als zu favorisierenden Impfstamm** über die durch i.p. und orale Immunisierung vermittelte gleichhohe Schutzwirkung gegen eine Toxoinfektion;
(im Hinblick auf die sinngemäße Übertragung dieser Gesetzmäßigkeiten auf weitere "Enteritidis"-Salmonellen) :

    **a) Vortest zum Nachweis der extrem differierenden Empfänglichkeit von Kücken/Hühnern und Mäusen für Salmonella typhimurium durch Bestimmung der i.p. $LD_{50}$-Werte von S.tm Wildstamm und S.tm Nal 2/Rif 9 Impfstamm für Kücken und Mäuse**
       Die unterschiedlichen i.p. $LD_{50}$-Werte für Mäuse und Zwei-Tage Kücken von S.tm Wildstamm und dem S.tm Nal 2/Rif 9 Impfstamm sowie zusätzlich der i.p. $LD_{50}$-Wert des Wildstammes für Kücken im Alter von 17 Tagen, sind in Tabelle 1 dargestellt.

Tabelle 1

| S.tm Wildstamm und S.tm Nal 2/Rif 9 Impfstamm: Vergleichende i.p. $LD_{50-}$ Werte bei Mäusen und Kücken | | | |
|---|---|---|---|
| S.tm | Zwei-Tage Kücken (cfu) | 17-Tage Kücken (cfu) | ICR Mäuse (cfu) |
| Wildstamm | $10^6$ | $10^8$ | $10^1$ |
| Nal 2/Rif 9 | $10^7$ | n.t. | $10^6$ |

Die $LD_{50}$-Werte für Kücken und Mäuse in Tabelle 1 verdeutlichen die geringere Empfänglichkeit der Kücken für S.tm, welche durch einen geringeren Attenuierungsgrad der Impfstämme kompensiert werden muß.

**b) Erkennung des für Kücken/Hühner optimal attenuierten Salmonella typhimurium Impfstammes (ohne und mit Rtt-Marker als Impfstamm-optimierende Envelope-Mutation) über die durch i.p. und orale Immunisierung vermittelte gleichhohe Schutzwirkung gegen eine Toxoinfection**

Als Kriterium "**optimale Attenuierung**" dienten die durch **einmalige**

- **i.p. Immunisierung** am zweiten Tag nach dem Schlüpfen mit dem Impfstamm S.tm Nal 2/Rif 9 und dem für Kücken überattenuierten Stämmen S.tm Pur⁻ (i.p. $LD_{50}$ Maus $10^{7.5}$ cfu) und Zoosaloral (i.p. $LD_{50}$ $10^{8.2}$ cfu): siehe Tabelle 2

- **orale Immunisierung** binnen ≤ 36 Stunden oder am vierten Tag nach dem Schlüpfen mit S.tm Nal 2/Rif 9; S.tm Nal 2/Rif 9/Rtt; sowie dem für Kücken überattenuierten Kälber Impfstoff Zoosaloral: siehe Tabelle 3

**erreichbaren gleichen Schutzwerte** gegen eine zwei Wochen später erfolgende etwa $LD_{75}$-Toxoinfektion.

Tabelle 2

| Erreichbare Immunität gegen eine Toxoinfektion **bei einmaliger i.p. Immunisierung am zweiten Lebenstag mit $10^6$ cfu** von Mutanten unterschiedlicher Attenuierungshöhe; Belastung am 16. Lebenstag mit i.p. $3 \times 10^8$ cfu des Wildstammes (Mittelwert aus drei Versuchen) | |
|---|---|
| i.p.Immunisierung:$10^6$ cfu | |
| S.tm-Impfstamm/Teststamm | i.p. Belastung Letalität (%) |
| Nal 2/Rif 9 | 25 |
| Pur⁻81 | 25 |
| Zoosaloral | 30 |
| Kontrolle | 75 |

Tabelle 3

| Erreichbare Immunität gegen eine Toxoinfektion **bei einmaliger oraler Immunisierung mit $10^9$ cfu** der Impfstämme und dem für Kücken, überattenuierten Zoosaloral; Belastung am 16.Lebenstag mit i.p. $3 \times 10^8$ cfu S.tm Wildstamm (Mittelwert aus vier Versuchen) | | | |
|---|---|---|---|
| | orale Immunisierung am | | |
| S.tm-Impfstamm/ Teststamm | mit (cfu) | 2.d i.p. Letalität | 4.d Belastung (%) |
| Nal 2/ Rif 9 | $10^9$ | 23 | 35 |
| | $10^8$ | 27 | n.t. |
| Nal 2/ Rif 9 Rtt | $10^9$ | 24 | 40 |
| | $10^8$ | 26 | n.t. |

Tabelle 3   (fortgesetzt)

| Erreichbare Immunität gegen eine Toxoinfektion **bei einmaliger oraler Immunisierung mit $10^9$ cfu** der Impfstämme und dem für Kücken, überattenuierten Zoosaloral; Belastung am 16.Lebenstag mit i.p. $3 \times 10^8$ cfu S.tm Wildstamm (Mittelwert aus vier Versuchen) | | | |
|---|---|---|---|
| | orale Immunisierung am | | |
| S.tm-Impfstamm/ Teststamm | mit (cfu) | 2.d i.p. Letalität | 4.d Belastung (%) |
| Zoosal- oral | $10^9$ $10^8$ | 42 47 | 60 65 |
| Kontrolle | | 75 | |

Die Tabelle 2 zeigt, daß die einmalige i.p. Immunisierung mit allen Impfstämmen die Letalität einer unphysiologischen Toxoinfektion von etwa 75 % auf ≤ 30 % senkt.

Wie Tabelle 3 ausweist, wird diese Letalitätssenkung — im Gegensatz zum Zoosaloral und Stoffwechseldrift-Mutanten mit Generationszeiten ≥ 33 Minuten und i.p. $LD_{50} \geq 10^{6.5}$ cfu — auch durch die einmalige orale Immunisierung mit dem Impfstamm S.tm Nal 2/Rif 9 (ohne und mit Rtt-Marker) erreicht. D.h. dieser Impfstamm ist demzufolge für Kücken optimal attenuiert.

Weitere Informationen der Tabelle 3 zeigen die

- Überattenuierung des Kälberimpfstoffes Zoosaloral
- bezüglich des Schutzes gegen eine Toxoinfektion weniger effektive Immunisierung am vierten Lebenstag. Dies ist vermutlich Folge der am vierten Lebenstag höheren Kolonisationsresistenz, welche die Translokations- und Penetrationsraten der Impfstämme beeinflussen dürfte.

**Beispiel 5**

**S.tm, S.ent, S.inf und S.ana: Isolierung von spontanen Antibiotika-Resistenz-Klonen als** (Ein- und) **Zweimarker Impfstämme mit optimaler Attenuierung für Kücken/Hühner mit Hilfe des "Attenuierungsäquivalenz** (verlängerte) **Generationszeit"** (siehe auch Beispiel 1)

$10^9$ bis $10^{10}$ cfu der Wildstämme werden auf Nähragar mit 400 µg Streptomycin/ml gespatelt und etwa zwei Tage bei 37°C incubiert. Die Resistenz-Klone werden über Nähragar passagiert, anschließend auf erhaltene Resistenz kontrolliert, im Vortest die im Vergleich zum Wildstamm weniger oder mehr verminderte Extinktion (Spekol mit Röhrchenansatz, Zeiss-Jena, Wellenlänge 650 nm, Startkeimzahl $10^7$ cfu, Bebrütung im Schüttel-wasserbad für drei Stunden bei 37°C) bestimmt (siehe Beispiel 1) und bei geeignet erscheinenden Klonen die Generationszeit mittels des Abbott MS-2 Testsystems gemessen. Von Klonen mit um drei bis sechs Minuten verlängerter Generationszeit werden im zweiten Schritt sinngemäß wie oben beschrieben Rifampicin-Resistenz-Klone (Nähragar mit 400 µg Rifampicin/ml) gewonnen, von diesen die Generationszeit bestimmt und Sm/Rif-Klone mit einer Generationszeit von etwa 31 bis 32 Minuten als Zweimarker-Impfstämme favorisiert. (Die Verwendung der Sm-Stwd-Attenuierung anstelle der Nal-Stwd ist vorteilhaft, da der Ssq-Marker (siehe Beispiel 5) als Envelope-Mutation — in z.B. Sm/Rif Impfstämmen (bei Verzicht auf eine Nal-Stwd-Attenuierung als Gyrase-Mutation) — auch gegen das derzeit wirksamste Antibiotikum Ciprofloxacin eine Supersensibilität vermittelt).

**Beispiel 6**

**Zusätzlicher Einbau eines Impfstamm-optimierenden/-Akzeptanz-erhöhenden Ssq("Sicherheits-/ und Therapie")-Markers in die Wild- bzw. Impfstämme**

Eine frische Kultur wird in PBS suspendiert und mit 100 µg/ml N-Methyl-N -Nitro-N-Nitrosoguanidin (MNG, ZIMET-Jena) bis zur Überlebensrate von etwa 10 % behandelt. Nunmehr wird für zwei Stunden bei 37°C in Nährbouillon mit 0,4 µg Chloramphenicol bebrütet und nachfolgend die klassische Behandlung mit 1000 IU Penicillin/ml durchgeführt. Durch Stempeltechnik werden Klone mit fehlendem Wachstum auf Nähragar mit 0,4 µg Chloramphenicol (0,01 µg Ciprofloxacin, 1,0 µg Doxycyclin)/ml gewonnen. Als **Ssq** (**S**uper**s**ensitivity to **q**uinolones)-Stämme werden solche Klone definiert und als Impfstamm-optimierender/-Akzeptanz-erhöhender Sicherheits- und Therapie-Marker genutzt, welche

- auf Nähragar mit 0,4 µg Chloramphenicol, **0,01 µg Ciprofloxacin** oder 1,0 µg Doxycyclin (sowie meist 30 µg Erythromycin)**/ml nicht wachsen**
- eine anzustrebende Reversionshäufigkeit von $\leq 10^{-7}$ aufweisen.

Die Reversionshäufigkeit der Envelope-Mutation wird besser auf Nähragar mit 20 oder 30 µg Erythromycin/ml bestimmt, da bei den hohen Keimzahlen sich auf Ciprofloxacin, Chloramphenicol oder Doxycyclin das Restwachstum zu einer um etwa zwei Stufen höheren Konzentration hin verschiebt.

Klone mit Ssq-Marker, welche durch Mutagen-Behandlung keine oder eine nur unwesentliche, Ko-Mutation bedingte, Generationszeitverlängerung/ Attenuierung erfahren, sind als Impfstämme geeignet.

**Beispiel 7**

**Erkennung der für Kücken/Hühner optimal attenuierter S. typhimurium, S. enteritidis, S. infantis und S. anatum Impfstämme über den Nachweis der erhaltenen bzw. restlichen Invasivität der Stoffwechseldrift** (Stwd) Einmarker-Mutanten und **Zweimarker-Impfstämme für Kücken**

$\leq$ 36 Stunden alte Kücken werden mit $10^9$ cfu der jeweiligen Wildstämme, Stwd-Einmarker-Mutanten bzw. Stwd-Zweimarker-Mutanten oral infiziert. Nach fünf und acht Tagen werden die Kücken getötet, die steril entnommene Leber homogenisiert, auf Nähragar ausgestrichen und das restliche Material mit Nährbouillon versetzt. Die gewachsenen Kolonien bzw. Kulturen werden auf 0-Gruppen-Identität und Marker geprüft.

Nach fünf Tagen liegen bei S.typhimurium und S.enteritidis die Keimzahlen/Gramm Leber in der Größenordnung um $10^3$ cfu, nach acht Tagen im Bereich um $10^2$ cfu. Demgegenüber bewegen sich nach fünf Tagen bei S.infantis und S.anatum die Keimzahlen im Grenzbereich der quantitativen Erfassung um $10^1$ bis $\leq 10^2$ cfu und nach acht Tagen gelingt der Nachweis oft nur noch über die Anreicherung. Diese für S.infantis und S.anatum gefundenen niedrigeren Keimzahlen/Gramm Leber korrelieren offensichtlich mit der von Methner, U. (Dissertation Universität Leipzig, Veterinärmedizinische Fakultät, 1991) berichteten Beobachtung, daß S.infantis für Hühner vermindert invasiv ist.

Die generell erhaltene Invasivität der favorisierten Impfstämme (und Einmarker-Mutanten), welche — gemessen an den angezüchteten Koloniezahlen — nicht ganz die Werte der homologen Wildstämme erreicht, ist für den Impferfolg offensichtlich essentiell (Barrow, P.A. et al. Res. Microbiol., 1990, **141**, 851-853).

**Beispiel 8**

**Ermittlung der prozentualen Häufigkeit und Dauer der Ausscheidung des Impfstammes S.tm Nal 2/Rif 9 ohne und mit Rtt-Marker nach oraler Immunisierung von $\leq$ 36 Stunden alten Kücken und des S.tm Nal 2/Rif 9 nach oraler Immunisierung von vier Tage alten Kücken:**

Die prozentuale Häufigkeit in den untersuchten Kotproben und die maximal nachweisbare Ausscheidungsdauer des Impfstammes ohne und mit Rtt-Marker in Abhängigkeit vom Alter der Kücken bei der oralen Immunisierung ist in Abbildung 1 aufgezeichnet.

Abbildung 1

Häufigkeit (Prozentsatz positiver Proben nach Anreicherung) und Dauer (letztmalig positiver Befund, Nachweisgrenze etwa 10 Keime/Gramm Kot) der Ausscheidung der Impfstämme S.tm Nal 2/Rif 9 ohne ( • — ) und mit Rtt-Marker ( ▲ — — ) nach einmaliger oraler Immunisierung < 36 Stunden alter Kücken mit $10^9$ cfu sowie mit S.tm Nal 2/Rif 9 (Einzel-Meßwerte nicht eingetragen, ( — )) am vierten Lebenstag. (Mittelwerte aus drei bis fünf Versuchen)

Prozentualer Anteil positiver Kotproben / Tage nach Immunisierung

Der Abbildung 1 sind folgende Fakten zu entnehmen

- Bei Immunisierung binnen ≤ 36 Stunden nach dem Schlüpfen wird der S.tm Nal 2/Rif 9 über die geprüften 32 Tage hinaus ausgeschieden.
 Nach Einbau des Rtt-Markers wird drei Wochen nach der Immunisierung der Impfstamm nur noch selten nachgewiesen.
- Bei der Immunisierung am vierten Tag wird der S.tm Nal 2/Rif 9 (ohne Rtt-Marker), offensichtlich bedingt durch die in diesem Alter bereits vorhandene, über Anaerobier vermittelte Kolonisationsresistenz, nach dem 18. Tag nur noch gelegentlich nachgewiesen.
- Analog hierzu ist die prozentuale Häufigkeit positiver Anreicherungskulturen. Bezogen auf die orale Immunisierung binnen ≤ 36 Stunden gelingt der Nachweis von S.tm Nal 2/Rif 9 am 13. Tag noch in etwa 90 % der Proben, beim mit dem Rtt-Marker optimierten Impfstamm sind nur noch etwa 40 % der Kotproben positiv.

**Die Kolonisierungsdynamik der als Impfstämme favorisierten S.tm Ssq/ Sm 60/Rif 42, S.ent Ssq/Sm 24/Rif 12, S.inf Ssq/Sm 153/Rif 7 und S.ana Ssq/Sm 81/Rif 21** wurde dadurch charakterisiert, daß, nach oraler Applikation von $10^9$ cfu binnen ≤ 36 Stunden nach dem Schlüpfen, der Anteil des jeweiligen Salmonella-Impfstammes an der Enterobakterien-Flora in den abgesetzten Kotproben über einen Zeitraum von zwei Wochen untersucht wurde. Dabei **zeigten** die favorisierten Impfstämme (s.o.) **ein dem Impfstamm S.tm Nal 2/Rif 9/Rtt vergleichbares Ausscheidungsverhalten.**

**Beispiel, 9**

**Reduktion der quantitativen Ausscheidung homologer neutral Nal/Sm markierter Wildstämme bei immunisierten Kücken im Vergleich zu Kontrollen**

Die im Alter von ≤ 36 Stunden mit den Impfstämmen S.tm Nal 2/Rif 9/Rtt, S.tm Ssq/Sm 60/Rif 42, S.ent Ssq/ Sm 24/Rif 12, S.inf Ssq/Sm 153/Rif 7 bzw. S.ana Ssq/Sm 81/Rif 21 monovalent oral immunisierten Kücken wurden parallel zu gleichaltrigen Kontrollen am 17. Lebenstag mit $10^6$ (teilweise $10^7$) cfu des jeweils homologen Wildstammes infiziert.

Im Vergleich zu den Kontrollen scheiden die immunisierten Kücken den Wildstamm, — in Übereinstimmung zu den mit dem S.tm Prototyp-Impfstamm Nal 2/Rif 9 erzielten Ergebnissen —, insbesondere in den ersten fünf bis zehn Tagen nach dem oralen Challenge, deutlich vermindert aus (gemessen am Promille-Anteil der Enterobakterien-Besiedlungsdichte).

Die Differenzen betragen (mit der Zeit abnehmend) bis zu zwei Zehnerpotenzen. Ab sechsten bis zehnten Tag nach der oralen Belastung gleichen sich die Salmonellen-Besiedlungsdichten bei den immunisierten Kücken denen von den Kontrollen (im 0,1-Promille-Bereich der Enterobakterien-Keimzahlen) an. In Einzelfällen zeigen allerdings immunisierte Kücken eine verminderte Ausscheidung in der Größenordnung von einer log-Stufe auch noch nach dem zehnten Tag.

Eine vollständige Eliminierung der Salmonellen beim infizierten Einzeltier ist kaum zu erwarten, da, bis auf S.gallinarum pullorum, diese Erreger bei Hühnern sich offensichtlich wie eine "Normalflora" verhalten.

Die stark reduzierte Ausscheidung bei immunisierten Kücken/Hühnern, in Verbindung mit Hygienemaßnahmen, dürfte jedoch mittelfristig zu Salmonella-freien Hühnerbeständen führen.

**Beispiel 10**

**Abtrennung der Impfstämme von Wildstämmen:**

Die Abtrennung von Wildstämmen erfolgt beim

- S.tm Ssq/Sm 60/Rif 42, S.ent Ssq/Sm 24/Rif 12, S.inf Ssq/Sm 153/Rif 7 und S.ana Ssq/Sm 81/Rif 21 durch ihre Resistenz gegen 100 μg Rifampicin und 200 μg Streptomycin/ml Nähragar sowie das fehlende Wachstum auf Nähragar mit 0,4 μg Chloramphenicol (oder 0,01 μg Ciprofloxacin, 1,0 μg Doxycyclin bzw. 30 μg Erythromycin)/ml.
- S.tm Nal 2/Rif 9/Rtt durch seine Resistenz gegen 100 μg Rifampicin und 12,5 μg Nalidixinsäure * /ml Nähragar sowie durch das fehlende Wachstum auf Nähragar mit 30 μg Erythromycin/ml.

**Beispiel 11**

**Massenkultur, Präparation und Anwendung der Impfstämme:**

Zur Herstellung der Lebendimpfstoffe werden die (Zweimarker-Impfstämme ohne Ssq-Marker oder)Dreimarker-Impfstämme mit Ssq-Marker in einem geeigneten Vollmedium als Flüssigkultur bis zum Ende der logarithmischen Phase gezüchtet. Die Bakteriensuspensionen werden mit einem üblichen Stabilisator versetzt und lyophilisiert.

Die so erhaltenen Impfstoffe werden in der Regel als einmalige orale Dosis von $10^8$ bis $10^9$ cfu an ≤ 36 Stunden alte Kücken verabfolgt. Hühner erhalten vor der Legeperiode eine in der Regel einmalige Immunisierung/Boosterung oral mit $10^9$ cfu parenteral mit etwa $10^8$ cfu.

**Patentansprüche**

1. Salmonella-Lebendimpfstoff, hergestellt aus mindestens einem attenuierten immunogenen Lebendimpfstamm, der zur Attenuierung zumindest eine Streptomycin- und eine Rifampicin-Resistenz Stoffwechseldrift-Mutation aufweist, **dadurch gekennzeichnet, daß** ein Envelope-Marker dem Lebendimpfstamm eine erhöhte Sensibilität gegenüber einem Antibiotikum aus der Gruppe der Chinolone, Chloramphenicole oder Tetracycline verleiht und der Lebendimpfstamm neben der Streptomycin- und der Rifampicin-Resistenz keine die erhöhte Sensibilität gegenüber den vorgenannten Antibiotika ausschließende Antibiotika-Resistenzmutation aufweist.

2. Lebendimpfstoff nach Anspruch 1, **dadurch gekennzeichnet, daß** der Envelope-Marker dem Impfstamm eine erhöhte Sensibilität gegenüber dem Antibiotikum Ciprofloxacin verleiht.

3. Lebendimpfstoff nach Anspruch 1 oder 2, **dadurch gekennzeichnet, daß** er mindestens einen Impfstamm eines Serovars der O-Gruppen B, z.B. Salmonella typhimurium, D, z.B. Salmonella enteritidis, C, z.B. Salmonella infantis, und E, z.B. Salmonella anatum, als Mono-, Bi-, Tri- oder Tetra-Vakzin enthält.

4. Lebendimpfstoff nach Anspruch 3, **dadurch gekennzeichnet, daß** die

- Mono-Vakzine aus dem Impfstamm S.tm Sm 60/Rif 42/Ssq mit der Hinterlegungs-Nr. DSM 8433 oder der Impfstamm S.ent Sm 24/Rif 12/Ssq mit der Hinterlegungs-Nr. DSM 8435 besteht,
- Bi-Vakzine aus dem obigen S.tm Impfstamm oder S.tm Nal 2/Rif 9/Rtt mit der Hinterlegungs-Nr. DSM 8432 und dem S.ent Sm 24/Rif 12/Ssq bestehen,
- Tri- oder Tetra-Vakzine wahlweise aus einem der obigen S.tm Impfstämme; S.ent Sm 24/Rif 12/Ssq; S.inf Sm

* (Die geringere Nalidixinsäure-Resistenz des Rtt-Stammes gegenüber dem Nal 2/Rif 9 Ausgangsstamm ist Folge der die Permeabilität der outer membrane veränderten (nachträglich eingebauten) Rtt-Mutation).

153/Rif/Ssq mit der Hinterlegungs-Nr. DSM 8434 und/oder S.ana Sm 81/Rif 21/Ssq mit der Hinterlegungs-Nr. DSM 8441 bestehen.

**5.** Lebendimpfstoff zur oralen Immunisierung von Küken sowie oralen oder parenteralen Immunisierung/Boosterung von Hühnern gegen Salmonella-Infektionen nach einem der Ansprüche 1 bis 4, **dadurch gekennzeichnet, daß** der zur Herstellung ausgewählte Impfstamm eine Generationszeit von etwa 29 bis 34 Minuten hat.

**Claims**

**1.** A salmonella live vaccine produced from at least one attenuated immunogenic live vaccine strain comprising at least one streptomycin and one rifampicin resistance metabolism drift mutation for the attenuation, **characterized in that** an envelope marker provides an increased sensitivity of the live vaccine strain towards an antibiotic of the group quinolones, chloramphenicols or tetracyclins and **in that** the live vaccine strain comprises besides the streptomycin and the rifampicin resistance no antibiotic resistance mutation excluding the increased sensitivity towards said antibiotics.

**2.** The live vaccine according to claim 1, **characterized in that** the envelope marker provides an increased sensitivity toward the antibiotic Ciprofloxacin for the vaccine strain.

**3.** The live vaccine according to claim 1 or 2, **characterized in that** it comprises at least one vaccine strain of a serovar of the O-groups B, e.g. salmonella typhimurium, D, e.g. salmonella enteritidis, C, e.g. salmonella infantis, and E, e.g. salmonella anatum, as mono-, bi-, tri- or tetra-vaccine.

**4.** The salmonella live vaccine according to claim 3, **characterized in that**

- mono-vaccine either consist of the vaccine strain S.tm Sm 60/Rif 42/Ssq having deposit no. DSM 8433 or of the vaccine strain S.ent Sm 24/Rif 12/Ssq having deposit no. DM 8435,
- bi-vaccines consist of the above-mentioned S.tm vaccine strain or S.tm Nal 2/Rif 9/Rtt having deposit no. DSM 8432 and of S.ent Sm 24/Rif 12/Ssq,
- tri- or tetra-vaccines optionally consist of one of the above-mentioned S.tm vaccine strains; S.ent Sm 24/Rif 12/Ssq; S.inf Sm 153/Rif/Ssq having deposit no. DSM 8434 and/or S.ana Sm 81/Rif 21/Ssq having deposit no. DSM 8441.

**5.** Live vaccine for the oral immunization of chicks as well as for the oral or parenteral immunization/boostering of chickens against salmonella infections according to one of the claims 1 to 4, **characterized in that** the vaccine strain selected for the production has a generation time of about 29 to 34 minutes.

**Revendications**

**1.** Vaccin vivant de Salmonella, préparé à partir d'au moins une souche immunogène atténuée de vaccin vivant, qui présente pour l'atténuation au moins une mutation par déplacement de métabolisme de la résistance à la streptomycine et à la rifampicine, **caractérisé en ce qu'**un marqueur d'enveloppe confère à la souche de vaccin vivant une sensibilité accrue par rapport à celle d'un antibiotique du groupe des quinolones, des chloramphénicols ou des tétracyclines, et que la souche de vaccin vivant ne présente, outre la résistance à la streptomycine et à la rifampicine, aucune mutation de résistance aux antibiotiques qui exclurait la sensibilité accrue vis-à-vis des antibiotiques mentionnés ci-dessus.

**2.** Vaccin vivant selon la revendication 1, **caractérisé en ce que** le marqueur d'enveloppe confère à la souche de vaccin une sensibilité accrue par rapport à l'antibiotique ciprofloxacine.

**3.** Vaccin vivant selon la revendication 1 ou 2, **caractérisé en ce qu'**il contient au moins une souche de vaccin d'un sérovar des groupes O B, par exemple Salmonella typhimurium, D, par exemple Salmonella enteritidis, C, par exemple Salmonella infantis et E, par exemple Salmonella anatum, sous forme d'un mono-, d'un bi-, d'un tri- ou d'un tétra-vaccin.

**4.** Vaccin vivant selon la revendication 3, **caractérisé en ce que**

- les monovaccins sont obtenus à partir de la souche S.tm Sm 60/Rif 42/Ssq, avec le numéro de dépôt DSM 8433, ou la souche S.ent Sm 24/Rif 12/Ssq, portant le numéro de dépôt DSM 8435,
- les bivaccins sont obtenus à partir de la souche S.tm ci-dessus ou de S.tm Nal 2/Rif 9/Rtt, portant le numéro de dépôt DSM 8432, et S.ent Sm 24/Rif 12/Ssq,
- les tri- ou tétravaccins sont obtenus au choix à partir de l'une des souches de vaccin S.tm ci-dessus ; S.ent Sm 24/Rif 12/Ssq ; S.inf Sm 153/Rif/Ssq portant le numéro de dépôt DSM 8434 et/ou S.ana Sm 81/Rif 21/Ssq portant le numéro de dépôt DSM 8441.

5. Vaccin vivant pour immunisation orale de poussins, et aussi pour immunisation/immunisation de rappel orale ou parentérale de poules contre des infections à Salmonella selon l'une des revendications 1 à 4, **caractérisé en ce que** la souche de vaccin choisie pour la préparation a un temps de génération d'environ 29 à 34 minutes.